# EUROPEAN PATENT APPLICATION

(11) **EP 3 542 866 A1**
(43) Date of publication of application: **25.09.2019**
(21) Application number: 19160154.1
(22) Date of filing: 05.03.2013
(51) Int. Cl.: A61Q 19/00, A61Q 19/08, A61K 8/98

(54) **TOPICAL DERMAL FORMULATIONS AND METHODS OF PERSONALIZED TREATMENT OF SKIN**

(30) Priority: 07.03.2012 US 201261607883 P
(62) Divisional of application: 13710943.5
(71) Applicant: Fibrocell Technologies, Inc., Exton, PA 19341 (US)
(72) Inventor: PERNOCK, David M., Villanova, Pennsylvania 19085 (US)
(74) Representative: Newton, Michael Jonathan

(57) **Abstract**

Personalized skin-specific topical formulations containing conditioned medium obtained from cultures of fibroblasts has been developed. Unlike other topical formulations, these formulations are specific for the recipient-skin type specific, and conditioned medium is obtained from a subject with desired skin characteristics. For example, since African American skin is known to wrinkle less and later than Caucasian skin, conditioned medium may be obtained from African American skin for application to pale skin. In another embodiment, the fibroblasts are obtained from young skin for administration to older skin. In still another embodiment, the fibroblasts are obtained from skin that does not suffer from acne or discoloration, for application to skin that is prone to acne or discoloration. Examples of skin donor selection criteria include delayed wrinkling, small pores, resistance to sunburn, resistance to acne, uniform coloration or lack of blotching or age spots and good moisture retention.

## Description

### CROSS REFERENCE TO RELATED APPLICATIONS

This application claims benefit of U.S. Provisional Application No. 61/607,883 filed March 7, 2012, which is herein incorporated by reference in its entirety.

### FIELD OF THE INVENTION

The present invention is generally in the field of cell-derived cosmeceuticals for the personalized treatment of skin.

### BACKGROUND OF THE INVENTION

The skin is the largest organ in the body and its cosmetic appearance is one of the top priorities in most cultures. Skin has been classified into different skin types, which present with different responses to environmental abuses. The Fitzpatrick Skin Type classification often is used to define skin types. The scale ranges from type I (ivory white skin) to type VI (dark brown skin) and identifies skin type based on its reaction to UV light. Skin of color can be classified as skin types IV-VI.

The most obvious difference between skin types is skin coloration. Pigmentation in the skin is determined by melanocytes, the cells that make melanin, or pigment, in the skin. Melanocytes produce packages of melanin called melanosomes. Pointy extensions of the melanocytes then transfer melanosomes into the keratinocytes. Although the number of melanocytes among races varies, the differences which are not significant are present at birth. People generally have the same total number of melanocytes regardless of their race; it is the distribution of melanosomes in the keratinocytes that correlates with skin color. In white skin-typically types I-III-melanosomes are small and aggregated in complexes. In black skin-types V and VI-there are larger melanosomes that are distributed singly within keratinocytes. Rosdahl, et al., Acta Dermatovenereologica, 56(2): 159-61 (1979). The role of melanin in the skin is to absorb and scatter energy from UV light to protect the epidermal cells from damage. Melanin provides considerable protection from sun damage, and the degree of protection corresponds directly to the degree of pigmentation. This sun protection offers significant prevention of photo-aging, which is one of the primary cosmetic concerns in Fitzpatrick skin types I-III. Reviewed by Woolery-Lloyd, "Ethnic Skin Care, in November 2006 issue, Skin Inc. Magazine.

Skin quality deteriorates with age, injury, exposure to sun and other environmental agents, and, on occasion, disease or autoimmune disorders. The pathogenesis of skin aging is well defined; it is characterized by a decrease in collagen synthesis and an increase in collagen breakdown, mediated by metalloproteinases (Fisher, et al., Arch. Dermatol., 138(11):1462-70 (2002)). The aging process of the skin occurs as a result of both intrinsic and extrinsic factors. The factors that contribute to intrinsic or natural aging are both structural and functional. Structurally, the epidermis becomes thinner, the corneocytes are less adherent and the dermal-epidermal junction is flattened. Functionally, there is a reduction in the number and biosynthetic capacity of fibroblasts and the dermis becomes atrophic and relatively acellular and avascular. Exposure to ultraviolet light radiation is the primary cause of extrinsic or photoaging. Extrinsic aging characteristics are loss of elasticity, increased roughness and dryness, irregular pigmentation, deep wrinkling, a leathery appearance, blister formation and impaired wound healing. The visible appearance of aging, especially facial wrinkles and folds, are common effects that patients seek to reduce.

Although all skin types respond to environmental insults, studies have shown that different skin types respond differently. The cosmetic advantage of skin types IV-VI is an increased protection from photo-aging. However, the cosmetic disadvantage of skin of color is its propensity to develop hyperpigmentation or hypopigmentation, which ethnic clients frequently experience and are most concerned about preventing. Grimes, Dermatologic Clinics, 18(4):659-65 (2000). Induction of a hyperpigmentary response is thought to be through signaling by the protease-activated receptor-2 which together with its activating protease is increased in the epidermis of subjects with skin of color. Rawlings, Int. J. Cosmetic Science, 28:79-93 (2006).

Generally Caucasians have an earlier onset and greater skin wrinkling and sagging than other skin types. In general, increased pigmentary problems are seen in skin of color although one large study reported that East Asians living in the U.S.A. had the least pigment spots. Changes in skin biophysical properties with age demonstrate that the more darkly pigmented subjects retain younger skin properties compared with the more lightly pigmented groups. Reduced stratum corneum (SC) natural moisturizing factor levels have been reported in Asian skin, compared with Caucasian and skin in people of African origin. Reduced epidermal Cathepsin L2 levels in darker skin types reported in skin of African origin could contribute to the skin ashing problems. Increased pores size, sebum secretion and skin surface microflora, and increased mast cell granule size, occur in skin of African origin. The frequency of skin sensitivity is quite similar across different racial groups but the stimuli for its induction shows subtle differences. Several studies indicate that Asian skin maybe more sensitive to exogenous chemicals, probably due to a thinner SC and higher eccrine gland density. Reviewed in Rawlings, Int. J. Cosmetic Science, 28:79-93 (2006).

Options for the treatment of facial lines, wrinkles and folds include surgery, neurotoxins, fillers, lasers, non-ablative therapies, microdermabrasion and chemical peels. Many of these treatments vary in safety, efficacy, and duration of effect in the treatment of the signs of aging. A wide variety of materials have been applied to skin to combat environmental insults, ranging from mud and herbal mixtures, animal fats, to emulsions, lotions, creams, gels, and biologicals. Many pharmaceutically active agents have been mixed with lotions, gels, creams, solutions, and sprays for topical application. Examples include cortisone and antihistamines to decrease inflammation, antibiotics to treat infection, antifungals, anti-itch, and drying agents. Some include bleaching agents to treat aging spots and chemicals to remove hair. These formulations are very specific to particular active ingredients and do nothing to restore the skin quality or decrease the effects of aging.

More recently, topical biological formulations have been developed. For example, growth factors, peptide fragments, and other biologically active molecules are being incorporated into anti-aging cosmeceuticals, as described, for example, by Mehta, et al., J Drugs Dermatol., 7(9):864-71 (2008) and in US Publication No. 20090123503 by Naughton, et al. Living cells cultured *in vitro* secrete extracellular proteins and peptides, including growth factors, into the nutrient medium in which they are cultured. Medium exposed to cells in culture is referred to as "conditioned medium". Such conditioned media may be used to prepare growth factor-enriched cosmeceutical compositions as described in U.S. Patent No. 6,372,494.

While the use of growth factors to treat aging skin is gaining favor among skin care professionals, there remains an important and unmet need for more effective topical formulations for the treatment and/or prevention of skin damage, wrinkles and/or other defects due to aging and environmental factors, which is personalized for the ethnic and age differences in skin types.

It is therefore an object of the present invention to provide ethnic skin-specific topical formulations of conditioned medium for topical administration to patients for the prevention and reduction in signs of aging, and improvement in quality of skin.

It is also an object of the present invention to provide an ethnic skin-specific method for preventing or reducing signs of aging, and improvement in quality of skin.

### SUMMARY OF THE INVENTION

Personalized skin-specific topical formulations containing conditioned medium obtained from cultures of fibroblasts has been developed. Unlike other topical formulations, topical formulations are specific for the recipient-skin type specific, and conditioned medium is obtained from a subject with desired skin characteristics. In one embodiment, the fibroblasts are obtained by biopsy from an individual of the same ethnic background. In another embodiment, the fibroblasts are obtained from an individual of a different ethnic background. For example, since African American skin is known to wrinkle less and later than Caucasian skin, conditioned medium is obtained from African American skin for application to pale skin. In another embodiment, the fibroblasts are obtained from young skin for administration to older skin. In still another embodiment, the fibroblasts are obtained from skin that does not suffer from acne or discolorations, for application to skin that is prone to acne or discoloration. Example of skin donor selection criteria include delayed wrinkling, small pores, resistance to sunburn, resistance to acne, uniform coloration or lack of blotching or age spots and good moisture retention.

Preferred formulations include gels, creams, lotions, and ointments made from the conditioned medium obtained from culture of fibroblasts. Fibroblasts are obtained from a screened donor of a specific ethnic group, and expanded in culture.

The formulations may be mass-produced using a similar manufacturing process for use of the general population of a specific skin type. In this version of the product, a screened donor provides tissue for expansion of fibroblasts and creation of a master cell bank (MCB). After appropriate tests are conducted on the MCB, cells expanded from the master bank are used to create a working cell bank (WCB), which is in turn expanded for manufacture of conditioned media for use in the formulation of the allogeneic topical product. The manufacturing process is similar to the autologous process, has the same applications and all final formulations of the topical product are within the same concentration ranges.

The topical formulations of conditioned medium obtained by culturing dermal fibroblasts are topically administered to individuals for the prevention and/or reduction of signs of aging, wrinkling, blotching, loss of elasticity, dryness, age spots, and general improvement in quality of skin. In one embodiment, an ethnic skin type-specific formulation is administered to a subject of the same ethnicity. In another embodiment, an ethnic skin type-specific formulation is administered to a subject of different ethnicity and skin type. In these embodiments, the formulations are used to complement skin of a different ethnicity and/or type, conferring to the skin to which it is administered, favorable factors for example, conditioned medium from Caucasian skin (which exhibits small pores) can be administered to skin of African origin which has larger pore sizes. Similarly, conditioned media from akin of African origin (slow aging skin), can be applied to skin of Caucasian origin, to provide the benefits of the African skin i.e., slow aging.

### DETAILED DESCRIPTION OF THE INVENTION

### I. DEFINITIONS

"Amphiphilic" refers to a molecule combining hydrophilic and lipophilic (hydrophobic) properties.

"Cream" is used herein to refer to a viscous liquid or semi-solid emulsion of either the "oil-in-water" or "water-in-oil type".

"Fibroblasts" are specialized cells in the skin that produce collagen and other extracellular matrix components. They are the cells from which connective tissues develop and, as such, play critical roles in the development of human tissue, including the ability to synthesize extracellular matrix components that contribute to skin texture and the secretion of matrix fibers, including collagen. Collagen is a naturally occurring protein that constitutes one of the primary components of the dermis; it exists as a matrix of fibers that provides structure and support.

"Gel" is used herein to refer to a colloid in which the dispersed phase has combined with the continuous phase to produce a semisolid material, such as jelly.

"Hydrophilic" as used herein refers to substances that have strongly polar groups that readily interact with water.

"Hydrophobic" as used herein refers to substances that lack an affinity for water; tending to repel and not absorb water as well as not dissolve in or mix with water.

"Lipid Soluble" as used herein refers to substances that have a solubility of greater than or equal to 5g / 100ml in a hydrophobic liquid such as castor oil.

"Lotion" is used herein to refer to a low- to medium-viscosity liquid formulation.

"Ointment" is used herein to refer to a semisolid preparation containing an ointment base and optionally one or more active agents.

"Oil" is used herein to refer a composition containing at least 95% wt of a lipophilic substance. Examples of lipophilic substances include but are not limited to naturally occurring and synthetic oils, fats, fatty acids, lecithins, triglycerides and combinations thereof.

"Water Soluble" as used herein refers to substances that have a solubility of greater than or equal to 5g /100ml water.

### II. FORMULATIONS

The formulations are ethnic skin type-specific topical formulations for topical administration. The formulation contains conditioned culture media obtained by culturing biopsied fibroblasts in an excipient for topical administration. The fibroblasts are obtained from one or more individuals of a specific ethnic skin type, screened for disease and compatibility prior to culturing. The donor individuals are selected based on their specific ethnic skin types, and additionally, based on known desirable characteristics attributed to that skin type, for example, wrinkling, small pores, resistance to sunburn, resistance to acne, uniform coloration or lack of blotching or age spots and good moisture retention.

### A. Conditioned Cell Culture Medium Formulations

A suspension of fibroblasts is grown from a biopsy of donor skin using standard tissue culture procedures and used to prepare conditioned media for use in the topical formulations. Skin tissue (dermis and epidermis layers) is biopsied from a suitable donor's post-auricular area.

Allogeneic cell lines may also be mass-produced and expanded to create conditioned media for formulation of a mass-produced topical product, containing media from donor skin of a specific ethnic skin type, known to exhibit specific desired characteristics for example, wrinkling, small pores, resistance to sunburn, resistance to acne, uniform coloration or lack of blotching or age spots and good moisture retention.

The conditioned medium is preferably allogenic. The starting material and cellular expansion process to create the Master Cell Bank (MCB) for the allogeneic process is the same as the autologous process.

Once the media is sufficiently concentrated, powder or liquid is blended with the selected topical vehicle. Blending can occur manually or using a mechanical device. After formulation, the product is filled into an appropriate dispenser and shipped to the end user. Examples of final container may include a pump bottle, squeeze bottle, jar, tube or vial.

### i. Preparation of Cells

In order to produce the allogeneic fibroblast culture, donors are selected to provide starting tissue. Prior to collection of the biopsy skin tissue, the individual is given a general examination for good health and screened for blood borne pathogenic diseases, such as HIV and Hepatitis B. Once the donor qualifies for participation, biopsy samples may be collected and shipped as described for the autologous process above.

To provide conditioned media to a large population of customers, a MCB is first established for later cell expansion and media collection. To create the MCB, the biopsies collected from the donor are expanded using the autologous process described above. Once harvest is complete, a series of safety tests may be performed to ensure purity of the cell line, including the following:
Viral screening: Test for a panel of viral particles.
Sterility: Test for the absence of microorganisms.
Mycoplasma: Test specifically for absence of microorganisms classified as *Mycoplasma* species that are considered a potential contaminant in cell culture.
Endotoxin: Test for proteins causing a pyrogenic (fever) response.

In addition, perform efficacy tests to confirm the quality of the cells:
Cell Count: Quantification of cells in the harvested population.
Cell Viability: Percentage of viable cells in the population.
Identity: Percentage of cells determined to be fibroblasts.
Collagen Content: Amount of collagen present in the cell suspension, indicating a biologically active population of cells.

After final harvest, cells are aliquoted and stored cryogenically in the vapor phase of liquid nitrogen as described for the autologous process above. These cells represent the MCB, and are used downstream to seed additional cultures for conditioned media collection. Maintenance of multiple donor cell lines provide ongoing inventory for manufacturing.

Each MCB vial is capable of seeding a new fibroblast subculture line to create a Working Cell Bank (WCB). Fibroblasts will be passaged in conventional culture vessels to produce enough cells to adequately seed a large scale culture bioreactor. Vials harvested from the culture are frozen and placed into cryostorage to create the finalized WCB.

Frozen cells from the WCB are thawed and expanded using conventional cell culture. Cells are passaged until enough are created to seed a bioreactor. The Bioreactors are commonly used in the biotechnology industry to support the production of vaccines, antibodies and small molecules. To create a large amount of conditioned media for use in formulation of the topical product, a bioreactor may be used to produce a large scale culture to maximize the amount of media collected.

Adherent cell culture in a bioreactor is an existing technology that can be applied directly to this application. A number of off-the-shelf units exist in various sizes that constantly monitor culture conditions such as temperature, CO₂, pH and dissolved oxygen, ensuring consistency from lot to lot. Examples include:
CelliGen® Series (New Brunswick Scientific, Edison, NJ)
WAVE™ Bioreactor System (GE Healthcare, Piscataway, NJ)

Bioreactors employ microcarriers to act as the growth surface for adherent cells such as fibroblasts. The carriers are small 2D or 3D structure capable of supporting cell expansion directly to the surface. In large quantities, microcarriers provide a large amount of growth surface area for cells to attach within the bioreactor. Potential carriers include:
Poly blend such as BioNOC II® (Cesco Bioengineering, distributed by Bellco Biotechnology, Vineland, NJ) and FibraCel® (New Brunswick Scientific, Edison, NJ)
Gelatin, such as Cultispher-G (Percell Biolytica, Astrop, Sweden)
Cellulose, such as Cytopore™ (GE Healthcare, Piscataway, NJ) coated/uncoated polystyrene, such as 2D MicroHex™ (Nunc, Weisbaden, Germany), Cytodex® (GE Healthcare, Piscataway, NJ) or Hy-Q Sphere™ (Thermo Scientific Hyclone, Logan, UT)

Once seeded into a bioreactor containing microcarriers, the culture is fed with fresh media over the course of the process. Multiple feeds are performed during the culture every few days. The conditioned media is collected aseptically, aliquoted into appropriate containers and frozen for later processing.

Alternatively, classic culture vessels such as tissue flaks and Cell Stacks may be used to expand the WCB in place of bioreactors and collect media for use in the topical formulation.

### ii. Conditioned Media Characterization

### Total Collagen:

Testing for total collagen content is part of the release criteria injectable autologous cell therapy product, indicating that fibroblasts are biologically active in culture. Conditioned media has also been historically tested for collagen content as part of characterization testing. Testing can be conducted using the Sicrol Assay Kit (Biocolor Life Science Assays, United Kingdom). The kit measures collagen I-V and reports a total collagen content value.

### Amino Acids:

The IMDM component of Complete Growth Media contains amino acids in support of cellular expansion. Conditioned media samples collected as can be tested for amino acid content using known methods, for example, size exclusion chromatography.

### B. Carriers and Excipients

The carrier may be any gel, ointment, lotion, emulsion, cream, foam, mousse, liquid, spray, suspension, dispersion or aerosol which is capable of delivering actives from the cell culture medium to the tissue. In one embodiment, the carrier is a gel, which is odorless and tasteless and dissolves rapidly, such as a hydroalcoholic gel.

### Lotions

A lotion can contain finely powdered substances that are in soluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In one embodiment, the lotion is in the form of an emulsion having a viscosity of between 100 and 1000 centistokes. The fluidity of lotions permits rapid and uniform application over a wide surface area. Lotions are typically intended to dry on the skin leaving a thin coat of their medicinal components on the skin's surface.

### Creams

Creams may contain emulsifying agents and/or other stabilizing agents. In one embodiment, the formulation is in the form of a cream having a viscosity of greater than 1000 centistokes, typically in the range of 20,000-50,000 centistokes. Creams are often time preferred over ointments as they are generally easier to spread and easier to remove. The basic difference between a cream and a lotion is the viscosity, which is dependent on the amount/use of various oils and the percentage of water used to prepare the formulations. Creams are typically thicker than lotions, may have various uses and often one uses more varied oils/butters, depending upon the desired effect upon the skin. In a cream formulation, the water-base percentage is about 60-75 % and the oil-base is about 20-30 % of the total, with the other percentages being the emulsifier agent, preservatives and additives for a total of 100 %.

Examples of suitable ointment bases include hydrocarbon bases (e.g., petrolatum, white petrolatum, yellow ointment, and mineral oil); absorption bases (hydrophilic petrolatum, anhydrous lanolin, lanolin, and cold cream); water-removable bases (e.g., hydrophilic ointment), and water-soluble bases (e.g., polyethylene glycol ointments). Pastes typically differ from ointments in that they contain a larger percentage of solids. Pastes are typically more absorptive and less greasy that ointments prepared with the same components.

### Gels

Some emulsions may be gels or otherwise include a gel component. Some gels, however, are not emulsions because they do not contain a homogenized blend of immiscible components. Suitable gelling agents include, but are not limited to, modified celluloses, such as hydroxypropyl cellulose and hydroxyethyl cellulose; Carbopol homopolymers and copolymers; and combinations thereof. Suitable solvents in the liquid vehicle include, but are not limited to, diglycol monoethyl ether; alklene glycols, such as propylene glycol; dimethyl isosorbide; alcohols, such as isopropyl alcohol and ethanol. The solvents are typically selected for their ability to dissolve the drug. Other additives, which improve the skin feel and/or emolliency of the formulation, may also be incorporated. Examples of such additives include, but are not limited, isopropyl myristate, ethyl acetate, C12-C15 alkyl benzoates, mineral oil, squalane, cyclomethicone, capric/caprylic triglycerides, and combinations thereof.

### Foams

Foams consist of an emulsion in combination with a gaseous propellant. The gaseous propellant consists primarily of hydrofluoroalkanes (HFAs). Suitable propellants include HFAs such as 1,1,1,2-tetrafluoroethane (HFA 134a) and 1,1,1,2,3,3,3-heptafluoropropane (HFA 227), but mixtures and admixtures of these and other HFAs that are currently approved or may become approved for medical use are suitable. The propellants preferably are not hydrocarbon propellant gases which can produce flammable or explosive vapors during spraying. Furthermore, the compositions preferably contain no volatile alcohols, which can produce flammable or explosive vapors during use.

### Excipients

Appropriate excipients are selected based on the type of formulation. Standard excipients include gelatin, casein, lecithin, gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidol silicon dioxide, phosphates, sodium dodecyl sulfate, carboxymethylcellulose calcium, carboxymethylcellulose sodium, methylcellulose, hydroxyethylcellulose, hydroxypropylcellulose, hydroxypropylmethycellulose phthalate, noncrystalline cellulose, magnesium aluminum silicate, triethanolamine, polyvinyl alcohol, polyvinylpyrrolidone, sugars, and starches.

"Diluents" may be included in the formulations to dissolve, disperse or otherwise incorporate the carrier. Examples of diluents include, but are not limited to, water, buffered aqueous solutions, organic hydrophilic diluents, such as monovalent alcohols, and low molecular weight glycols and polyols (e.g. propylene glycol, polypropylene glycol, glycerol, butylene glycol).

"Emollients" are an externally applied agent that softens or soothes skin and are generally known in the art and listed in compendia, such as the "Handbook of Pharmaceutical Excipients", 4th Ed., Pharmaceutical Press, 2003. These include, without limitation, almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol and combinations thereof. In one embodiment, the emollients are ethylhexylstearate and ethylhexyl palmitate.

"Surfactants" are surface-active agents that lower surface tension and thereby increase the emulsifying, foaming, dispersing, spreading and wetting properties of a product. Suitable non-ionic surfactants include emulsifying wax, glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone and combinations thereof. In one embodiment, the non-ionic surfactant is stearyl alcohol.

"Emulsifiers" are surface active substances which promote the suspension of one liquid in another and promote the formation of a stable mixture, or emulsion, of oil and water. Common emulsifiers are: metallic soaps, certain animal and vegetable oils, and various polar compounds. Suitable emulsifiers include acacia, anionic emulsifying wax, calcium stearate, carbomers, cetostearyl alcohol, cetyl alcohol, cholesterol, diethanolamine, ethylene glycol palmitostearate, glycerin monostearate, glyceryl monooleate, hydroxpropyl cellulose, hypromellose, lanolin, hydrous, lanolin alcohols, lecithin, medium-chain triglycerides, methylcellulose, mineral oil and lanolin alcohols, monobasic sodium phosphate, monoethanolamine, nonionic emulsifying wax, oleic acid, poloxamer, poloxamers, polyoxyethylene alkyl ethers, polyoxyethylene castor oil derivatives, polyoxyethylene sorbitan fatty acid esters, polyoxyethylene stearates, propylene glycol alginate, self-emulsifying glyceryl monostearate, sodium citrate dehydrate, sodium lauryl sulfate, sorbitan esters, stearic acid, sunflower oil, tragacanth, triethanolamine, xanthan gum and combinations thereof. In one embodiment, the emulsifier is glycerol stearate.

"Buffers" are used to control pH of a composition. Preferably, the buffers buffer the composition from a pH of about 4 to a pH of about 7.5, more preferably from a pH of about 4 to a pH of about 7, and most preferably from a pH of about 5 to a pH of about 7. In a preferred embodiment, the buffer is triethanolamine.

"Preservatives" can be used to prevent the growth of fungi and microorganisms. Suitable antifungal and antimicrobial agents include, but are not limited to, benzoic acid, butylparaben, ethyl paraben, methyl paraben, propylparaben, sodium benzoate, sodium propionate, benzalkonium chloride, benzethonium chloride, benzyl alcohol, cetylpyridinium chloride, chlorobutanol, phenol, phenylethyl alcohol, and thimerosal.

"Penetration enhancers" are frequently used to promote transdermal delivery of drugs across the skin, in particular across the stratum corneum. A penetration enhancer may be added to enable the active agents to cross the barrier of the stratum corneum. Some penetration enhancers cause dermal irritation, dermal toxicity and dermal allergies. However, the more commonly used ones include urea, (carbonyldiamide), imidurea, N, N-diethylformamide, N-methyl-2-pyrrolidine, 1-dodecal-azacyclopheptane-2-one, calcium thioglycate, 2-pyyrolidine, N,N-diethyl-m-toluamide, oleic acid and its ester derivatives, such as methyl, ethyl, propyl, isopropyl, butyl, vinyl and glycerylmonooleate, sorbitan esters, such as sorbitan monolaurate and sorbitan monooleate, other fatty acid esters such as isopropyl laurate, isopropyl myristate, isopropyl palmitate, diisopropyl adipate, propylene glycol monolaurate, propylene glycol monooleatea and non-ionic detergents such as BRIJ® 76 (stearyl poly(10 oxyethylene ether), BRIJ® 78 (stearyl poly(20)oxyethylene ether), BRIJ® 96 (oleyl poly(10)oxyethylene ether), and BRIJ® 721 (stearyl poly (21) oxyethylene ether) (ICI Americas Inc. Corp.).

### III. Methods of Administration and Treatment

The formulations used herein may be used for local topical delivery to any location, especially areas in which the skin has thinned, discolored or wrinkled due to age. The methods are personalized, i.e., the source of the fibroblast conditioned medium is selected based on the need(s) of the recipient. In one embodiment, selection is ethnic skin type-specific, where the formulation is selected based on the recipient's specific skin type and the complementary characteristics present in a skin type of the same or different ethnicity. In one embodiment, the formulation is obtained from donor skin of the same ethnic skin type as the treated skin. In other embodiments the formulation is from a donor skin of a different ethnic skin type from the treated skin. The donor skin is selected based on the desired and missing characteristic of the recipient skin, which the donor skin is known to possess. Exemplary characteristics include delayed wrinkling, small pores, resistance to sunburn, resistance to acne, uniform coloration or lack of blotching or age spots and good moisture retention. For example, a formulation made from a donor skin with small pores can be applied to a recipient skin with large pores. Alternatively, a formulation made from a donor skin of with good genetics and characteristics for later onset of wrinkling can be applied to a recipient skin with early onset wrinkling.

It is not necessary to characterize the cell culture medium for the presence or absence, or quantity, of specific molecules. One only selects the source. It is believed that it is a combination of factors in the cell cultured medium which is responsible for providing the desired effect(s). The formulation may act by stimulating cells in the dermis to grow and divide, by increasing production of extracellular matrix components (e.g. collagen), and/or by stimulating the reorganization of existing extracellular matrix, which may have multifactorial effects for improvement of the skin.

Other aspects of the invention for which protection is sought are:
1. A personalized skin type-specific topical formulation comprising
   an excipient for topical application, and
   conditioned culture media obtained by culturing biopsied fibroblasts, wherein the fibroblasts are obtained from one or more individuals of a specific ethnic group, age, resistance to infection, resistance to discoloration, or skin quality, screened for disease and compatibility prior to culturing, wherein the excipient is blended with the conditioned culture media or materials therein to form the formulation.
2. The formulation of paragraph 1 selected from the group consisting of a gel, ointment, lotion, emulsion, cream, foam, mousse, liquid, spray, suspension, dispersion and aerosol.
3. The formulation of paragraph 1 wherein the fibroblasts have been passaged multiple times to produce the conditioned culture media.
4. The formulation of paragraph 3 wherein the fibroblasts are passaged after reaching 40% confluence.
5. The formulation of paragraph 1 wherein the formulation contains the material obtained from 1.5 liters of conditioned cell culture media where the cells are grown to at least 80% cell confluence.
6. A method of making a personalized skin type-specific topical formulation comprising
   selecting one or more individuals of a specific ethnic group, age, resistance to infection, resistance to discoloration, or skin quality,
   obtaining fibroblasts from the one or more individuals,
   culturing the fibroblasts in cell culture, and mixing the conditioned culture media, or materials therein, with the excipient to make a formulation for topical application.
7. The method of paragraph 6 wherein the one or more individuals are selected based on their ethnic skin type as characterized by the Fitzpatrick Skin Type classification.
8. The method of paragraph 6 wherein the skin type possesses one or more desired characteristics selected from the group consisting of wrinkling, small pores, resistance to sunburn, resistance to acne, uniform coloration or lack of blotching or age spots and good moisture retention.
9. The method of paragraph 6 wherein the fibroblasts are passaged after reaching 40% confluence.
10. The method of paragraph 6 wherein the autologous fibroblasts are obtained from three 3-mm punch skin biopsies.
11. The method of paragraph 6 wherein the formulation contains the material obtained from 1.5 liters of conditioned cell culture media where the cells are grown to at least 80% cell confluence.
12. The method of paragraph 6 wherein the conditioned cell culture media is dried to produce material which is blended with the excipient.
13. A personalized skin type-specific method for treating skin comprising topically administering formulations of conditioned medium obtained by culturing dermal fibroblasts obtained from one or more individuals of a specific ethnic group, age, resistance to infection, resistance to discoloration, or skin quality, to a skin in need thereof.
14. The method of paragraph 13 wherein a formulation from a donor skin is from a younger donor than the person with the treated skin.
15. The method of paragraph 14 wherein the donor has relatives with late or minimal onset of wrinkling.
16. The method of paragraph 13, wherein the donor skin exhibits desired characteristics selected from the group consisting of wrinkling, small pores, resistance to sunburn, resistance to acne, uniform coloration or lack of blotching or age spots and good moisture retention.
17. The method of paragraph 13 wherein a formulation is administered to a recipient skin of the same ethnic origin as the fibroblasts are derived from.
18. The method of paragraph 17 wherein the formulation the donor skin is of African origin and is administered to a skin of African origin.
19. The method of paragraph 13, wherein a formulation is administered to a recipient skin, and wherein the formulation is the formulation of any one of paragraphs 1 to 5.

## Claims

1. A method of making an ethnic skin type-specific topical formulation comprising:
determining personalized skin type needs of an intended recipient;
selecting based upon the needs of the intended recipient, one or more donors of a specific ethnic group that is known to possess the personalized skin type needs of the intended recipient, wherein the one or more donors are also selected based on wrinkling, small pores, resistance to sunburn, resistance to acne, uniform coloration, lack of blotching, lack of age spots, good moisture retention, resistance to infection, resistance to discoloration, or skin quality;
obtaining fibroblasts from the post-auricular area of one or more donors;
culturing the fibroblasts in cell culture to produce a conditioned culture media containing a biologically active amount of collagen content and a favorable factor that exhibits the personalized skin type needs of the intended recipient; and
mixing the conditioned culture media, or factors derived from the conditioned culture media, with an excipient to make the formulation.

2. The method of claim 1 wherein the one or more donors is selected based on ethnic skin type as **characterized by** the Fitzpatrick Skin Type classification.

3. The method of claim 2 wherein the skin type possesses one or more desired characteristics selected from the group consisting of minimal wrinkling, small pores, resistance to sunburn, resistance to acne, uniform coloration, lack of blotching or age spots and good moisture retention.

4. The method of claim 1 wherein the fibroblasts are passaged after reaching 40% confluence.

5. The method of claim 1 wherein the fibroblasts are obtained from three 3-mm punch skin biopsies.

6. The method of claim 1 wherein the conditioned culture media is obtained from 1.5 liters of conditioned cell culture media where the cells thereof are grown to at least 80% cell confluence.

7. The method of claim 1 wherein the conditioned culture media is dried to produce material which is blended with the excipient.

8. The method of claim 1 wherein the cell culture of fibroblasts is tested for a panel of viral particles, microorganisms, Mycoplasma species, and endotoxin.

9. A personalized skin type-specific method for treating skin type needs of a recipient comprising topically administering an ethnic skin type-specific formulation of conditioned medium from cultured dermal fibroblasts from the post-auricular area of one or more donors selected from a specific ethnic group that is known to possess the personalized skin type needs and selected for one or more factors of younger wrinkling, small pores, resistance to sunburn, resistance to acne, uniform coloration, lack of blotching, lack of age spots, good moisture retention, resistance to infection, resistance to discoloration, or high skin quality, to the skin of the recipient, wherein the conditioned medium contains a biologically active amount of collagen content and a favorable factor that exhibits the personalized skin type needs.

10. The method of claim 9 wherein the formulation is from the one or more younger donors than the intended recipient.

11. The method of claim 10 wherein the one or more donors have relatives with late or minimal onset of wrinkling.

12. The method of claim 9, wherein the one or more donors have skin that exhibits desired characteristics selected from the group consisting of minimal wrinkling, small pores, resistance to sunburn, resistance to acne, uniform coloration, lack of blotching or age spots and good moisture retention.

13. The method of claim 9 wherein the recipient is of the same ethnic origin as the one or more donors of the fibroblasts.

14. The method of claim 9 wherein the recipient is of a different ethnic origin as the one or more donors of the fibroblasts.

15. The method of claim 14 wherein the recipient is a Caucasian and the one or more donors are of African origin, wherein the one or more donors are selected for slow aging skin.
